(19) Europäisches Patentamt
European Patent Office
Office européen des brevets

(11) EP 1 159 954 A2

(12) EUROPEAN PATENT APPLICATION

(43) Date of publication:
05.12.2001 Bulletin 2001/49

(51) Int Cl.7: A61K 7/48

(21) Application number: 01401398.1

(22) Date of filing: 29.05.2001

(84) Designated Contracting States:
AT BE CH CY DE DK ES FI FR GB GR IE IT LI LU MC NL PT SE TR
Designated Extension States:
AL LT LV MK RO SI

(30) Priority: 31.05.2000 JP 2000161867

(71) Applicant: SHISEIDO COMPANY LIMITED
Chuo-ku, Tokyo 104-8010 (JP)

(72) Inventors:
- Yamasaki, Ryota, c/o Shiseido Research Center
Yokohama-shi, Kanagawa 224-8558 (JP)
- Yokotsuka, Akihito,
c/o Shiseido Research Center
Yokohama-shi, Kanagawa 224-8558 (JP)
- Kazama, Yukiko, c/o Shiseido Research Center
Yokohama-shi, Kanagawa 224-8558 (JP)

(74) Representative: Rinuy, Santarelli
14, avenue de la Grande Armée
75017 Paris (FR)

# (54) Solid water-in-oil type emulsion cosmetic composition

(57) A stick-type solid water-in-oil type emulsion cosmetic composition having an excellent stability, having a refreshing feeling and freshness after finish when coated on the skin and particularly giving a soft feeling of use when coated, which contains (a) 0.5 to 40.0% by weight of a hydrogenated jojoba oil, (b) 0.1 to 10.0% by weight of a surfactant composed of polyoxyalkylene-modified silicone or alkyl- and polyoxyalkylene-modified silicone and (c) 5.0 to 50.0% by weight of water.



Printed by Jouve, 75001 PARIS (FR)

## Description

BACKGROUND OF THE INVENTION

1. Field of the Invention

The present invention relates to a stick-type solid water-in-oil emulsion cosmetic composition. More specifically, it relates to a novel stick-type solid water-in-oil emulsion type cosmetic composition having an excellent stability, having a refreshing feeling and freshness after finish when coated on the skin, in spite of the solid form, and particularly giving a soft feeling of use when coated.

2. Description of the Related Art

In recent years, solid water-in-oil type emulsion cosmetic compositions having excellent stability have been appearing on the market. Particularly, this type cosmetic composition gives an emulsion type treatment, even in the form of a solid, and therefore, the effect on the skin is high. Further, since water is added, a novel feeling of use such as a refreshing feeling and feeling of wateriness can be obtained.

However, in most cases, a hydrocarbon-based wax having a high solidifying power is mainly used as a solid oil of the essential ingredient for solidification. This is not at all satisfactory in terms of the "softness to the skin".

SUMMARY OF THE INVENTION

Accordingly, an object of the present invention is to provide a novel stick-type solid water-in-oil type emulsion cosmetic composition which is capable of solving the above problem of the prior art and which is capable of providing a refreshing feeling and freshness after finish when coated on the skin, and particularly giving a soft feeling of use when coated.

In accordance with the present invention, there is provided a stick-type solid water-in-oil type emulsion cosmetic composition comprising the following components (a) to (c):

(a) 0.5 to 40.0% by weight of a hydrogenated jojoba oil;
(b) 0.1 to 10.0% by weight of a surfactant composed of polyoxyalkylene-modified silicone or alkyl- and polyoxyalkylene-modified silicone; and
(c) 5.0 to 50.0% by weight of water.

DESCRIPTION OF THE PREFERRED EMBODIMENT

The present invention will now be explained in more detail. In this specification and in the claims the singular forms "a", "an" and "the" include plural referents unless the context clearly dictates otherwise.

The present inventors engaged in intensive studies to solve this problem and, as a result, found that a stick-type solid water-in-oil type emulsion cosmetic composition containing a hydrogenated jojoba oil, as a solid oil, can solve the above problem whereby the present invention has been completed.

The stick-type solid water-in-oil type emulsion cosmetic composition of the present invention has the property of easily melting on the skin due to the fact that the wax used has a suitably low melting point and spreading over the skin smoothly, since the yield value thereof at the time of application is small. Further, since it is an emulsion base, blending of a moisture retaining ingredient becomes possible and formulations, considering the skin in the winter when the skin can easily become rough, become possible.

The stick-type solid water-in-oil type emulsion cosmetic composition of the present invention is intended to mean one which is filled into a stick container and usually solidifies, including paste form, without fluidity at the temperature range of, for example, 0°C to 50°C, in which the cosmetic composition is used. Among the stick-type water-in-oil type emulsion cosmetic compositions, those from which the content is sending out in use are preferable. The "stick-type container" used in the present invention typically in the form of a column or cylinder type or an elliptical column type having a size of 5 - 40 mm diameter, preferably 8 - 25 mm diameter especially for a foundation, 3 - 15 cm height, preferably 7 cm or more height for a foundation in view of the usability.

The hydrogenated jojoba oil used in the present invention is mainly a compound obtained from the hydrogenation of a jojoba oil mainly composed of an ester of a monovalent higher unsaturated fatty acid and monovalent higher unsaturated alcohol having the following chemical structure:

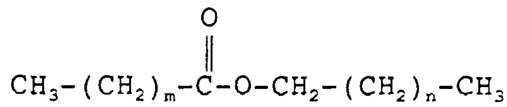

wherein m is 16, 18 or 20 and n is 18, 20 or 22, provided that the total carbon number is 38 - 44. As commercially available products, for example, partially hydrogenated jojoba oil such as Floraesters 20, Floraesters 30, and Floraesters 60 (all made by Floratech) and Jobacream M-40 (made by Koei Kogyosha) and completely hydrogenated jojoba oil such as Floraesters 70 and Floraesters 70 HG (all made by Floratech) and Extreme Hydrogenated Jojoba (made by Koei Kosansha) etc. are exemplified. Among these, it is preferable to use completely hydrogenated jojoba oil hydrogenating all unsaturated bond portions.

The amount of hydrogenated jojoba oil to be formulated is 0.5 to 40.0% by weight, particularly preferably 2.0 to 20.0% by weight, in the entire cosmetic composition.

The polyoxyalkylene-modified silicone or alkyl- and polyoxyalkylene-modified silicone can be represented by the following formula (I):

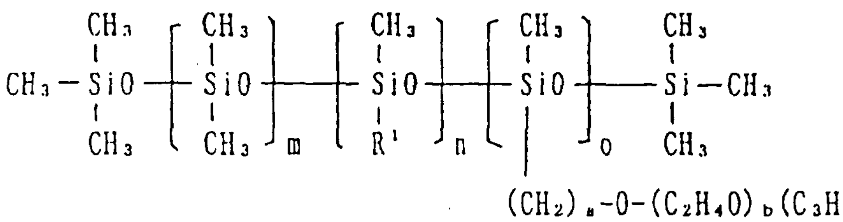

··· (I)

wherein a is an integer of 1 to 5, b is an integer of 7 to 100, c is an integer of 0 to 100, m is an integer of 10 to 500, n is an integer of 0 to 150, and o is an integer of 1 to 50, $R^1$ is a $C_1$ to $C_{26}$ alkyl group, and $R^2$ is a hydrogen atom or a $C_1$ to $C_5$ alkyl group.

The use of the polyoxyalkylene-modified silicone and alkyl- and polyoxyalkylene-modified silicone, are suitable for the reasons of, for example, fillability in containers, especially high temperature properties, usability, especially lack of stickiness, emulsion stability.

The amount of the surfactant formulated in the present cosmetic composition is selected, depending upon the amount of the aqueous phase emulsified, but 0.1 to 10.0% by weight, more preferably 1.0 to 8.0% by weight, in the total weight of the cosmetic composition is preferable.

The amount of the water formulated in the present cosmetic composition is 5.0 to 50.0% by weight, preferably 10.0 to 25.0% by weight, in the total cosmetic composition. When an amount of water is less than 5.0% by weight, a refreshing feeling at the time of use cannot be expected. In particular, for obtaining an extremely high refreshing feeling at the time of use, it is preferable to formulate at least 10.0% by weight. Further, the higher the amount of water formulated, the heavier the slip tends to become. Therefore, the amount of water formulated is preferably not more than 25.0% by weight.

In the present invention, as the wax ingredient, it is preferable to further include a hydrocarbon wax other than the hydrogenated jojoba oil. Examples of the hydrocarbon wax are ceresine wax, paraffin wax, microcrystalline wax, Fisher-Tropsh wax, SASOL wax, etc. Among these, the use of ceresine wax is preferable from the viewpoint of the uniform solidification in the container when filling. For instance, in the case of polyethylene wax alone, the filling property tends to become undesirable due to the relatively high melting point.

The ratio (i.e., ratio by weight) of the hydrogenated jojoba oil and the hydrocarbon wax is hydrocarbon wax/hydrogenated jojoba oil = 0.05 to 15, preferably 0.1 to 7.5. Here, an amount of hydrocarbon wax of not more than 15% by weight is preferable. Further, the total amount of the wax ingredient in the total weight of the cosmetic composition is preferably 3 to 22% by weight. The stability becomes further higher if hydrogenated jojoba oil is used in combination with hydrocarbon wax.

The solid water-in-oil type emulsion cosmetic composition of the present invention has a hardness at the time of measurement by a card tension meter of preferably 4 to 70 at 37°C, 200 g load, and 1.5ϕ. Particularly, a hardness of 7 to 40 is optimal from the viewpoint of the skin feeling.

Further, in the present invention, powders of pigments may be formulated, in addition to the above-mentioned essential ingredients of the hydrogenated jojoba oil, surfactant, and water. The pigment powder used in this case is not particularly limited so long as they are usually used in cosmetic compositions. It is possible to formulate powder of a pigment such as an inorganic pigment, organic pigment, or metal pigment. Examples of inorganic powders are, talc, kaolin, calcium carbonate, zinc white, titanium dioxide, red iron oxide, yellow iron oxide, black iron oxide, ultramarine,

titanium coated mica, bismuth oxichloride, Bengara, sintered pigments, Gunjo Pink, chromium hydroxide, titanium oxide coated mica, chromium oxide, aluminum cobalt oxide, Prussian Blue, carbon black, anhydrous silicic acid, magnesium silicate, bentonite, mica, zirconium oxide, magnesium oxide, zinc oxide, titanium oxide, light calcium carbonate, heavy calcium carbonate, light magnesium carbonate, heavy magnesium carbonate, calamine, etc. Here, a powder of a pigment which is treated to give it hydrophobicity is particularly preferable. The method of giving hydrophobicity may include treating of the surface with a high viscosity silicone, coating of the surface with a silicone resin reacted with an alkylhydrogen polysiloxane, further followed by treating with an alkene, treating with one or more types of a cationic activant, anionic activant, and nonionic activant, coating of the surface by a wax, etc. The surface should be made hydrophobic. The method is not particularly limited. Further, the amount of the pigment powder formulated is preferably not more than 50% by weight of the total cosmetic composition.

Further, the stick-type solid water-in-oil type emulsion cosmetic composition of the present invention may contain therein ingredients normally used for cosmetic compositions to an extent not detracting from the effects of the present invention. For example, the other surfactants such as those usually formulated into a cosmetic composition may be used, together with the above surfactant, irrespective of the ionic character thereof.

Examples of anionic surfactants are raw materials for soap; fatty acid soaps such as sodium laurate; higher alkyl sulfate ester salts such as sodium laurosulfate; alkyl ether sulfate ester salts such as polyoxyethylene (i.e., "POE") laurosulfate triethanol amine; N-acylsarcosine acids such as sodium lauroyl sarcosinate; higher fatty acid amide sulfonates such as sodium N-cocoyl-N-methyl taurate; phosphate ester salts such as POE stearyl ether phosphate; sulfosuccinates such as sodium di-2-ethylhexylsulfosuccinate; alkylbenzensulfonates such as sodium dodecylbenzensulfonate; N-acyl glutamates such as disodium N-stearoyl glutamate; higher fatty acid ester sulfate ester salts such as sodium hydrogenated glyceryl cocoate sulfate; sulfated oils such as Turkey red oil; POE alkyl ether carboxylic acid; POE alkylaryl ether carboxylate; $\alpha$-olefinsulfates; higher fatty acid ester sulfonates; secondary alcohol sulfate ester salts; higher fatty acid alkylolamide sulfate ester salts; sodium lauroyl monoethanolamide succinate; N-palmitoyl asparaginate ditriethanol amine; sodium caseine.

Examples of cationic surfactants are alkyl trimethyl ammonium salts such as stearyl trimethyl ammonium chloride; dialkyldimethyl ammonium salts such as distearyldimethyl ammonium chloride; alkyl pyridinium salts such as cetylpyridinium chloride; alkyl quaternary ammonium salts, alkyl dimethylbenzyl ammonium salts, alkyl isoquinolinium salts; dialkyl morphonium salts; POE alkyl amines; alkyl amine salts; polyamine fatty acid derivatives; amyl alcohol fatty acid derivatives; benzalkonium chloride, benzethonium chloride, etc.

Examples of amphoteric surfactants are imidazoline amphoteric surfactants such as sodium 2-undecyl-N,N, N-(hydroxyethylcarboxymethyl)-2-imidazoline; betaine surfactants such as lauryldimethyl-aminoacetate betaine, etc.

Examples of lyophilic nonionic surfactants are sorbitan fatty acid esters such as sorbitan monoisostearate and sorbitan sesquioleate; glyceryl polyglyceryl fatty acids such as glyceryl monostearate; propylene glycol fatty acid esters such as propylene glycol monostearate; hydrogenated castor oil derivatives; glyceryl alkyl ethers; polyoxyalkylene-modified silicone; alkyl- and polyoxyalkylene-modified silicone; etc.

Examples of hydrophilic nonionic surfactants are POE sorbitan fatty acid esters such as POE sorbitan monooleate; POE sorbite fatty acid esters such as POE-sorbitan monooleate; POE glyceryl fatty acid esters such as POE-glyceryl monoisostearate; POE alkyl ethers such as POE stearyl ethers and POE cholestanol ethers; POE alkyl phenyl ethers such as POE nonyl phenyl ethers; pluaronics such as Pluronic; POE-POP alkyl ethers such as POE·polyoxypropylene (i.e., "POP") cetyl ethers, tetra-POE·tetra-POP ethylene diamine condensation products such as Tetronic; POE castor oil, POE castor oil hydrogenated castor oil derivatives such as POE hydrogenated castor oil POE beeswax lanolin derivatives; alkanolamides; POE propylene glycol fatty acid esters; POE alkylamines; POE fatty acid amides; sucrose fatty acid esters; POE nonylphenyl formaldehyde condensation products; alkylethoxydimethylamineoxide; trioleylphosphoric acid, etc. However, please note that the present invention is not limited to the use of the above surfactants.

Further, these surfactants may be used alone or in any combinations thereof in the stick-type solid water-in-oil type emulsion cosmetic composition of the present invention.

The other ingredients include the aqueous phase ingredients, polyhydric alcohol, mucosaccharides (sodium hyaluronate etc.), organic acids and organic salts (amino acid, amino acid salts, oxyacid salts), and other moisture-retaining agents, alcohols such as ethanol, oil phase ingredients such as vaseline, solid and semisolid oils such as lanolin, silicone wax, fluorowaxes, silicone resins, silicone rubber, higher fatty acids, higher alcohols, liquid oils such as squalene, liquid paraffin, ester oils, triglyceride, volatile hydrocarbon oils, fluorocarbons, medicines such as vitamin E, vitamin E acetate, astringents, antioxidants, preservatives, fragrances, sodium phosphate (II), and other pH adjusters, clay minerals, thickners, UV blockers, etc. may be formulated in the solid water-in-oil type emulsion cosmetic composition of the present invention.

The stick-type solid water-in-oil type emulsion cosmetic composition of the present invention may be spread on the skin with the finger or a puff, or which can be directly spread on the skin from the stick. The stick-type solid water-in-oil type emulsion cosmetic composition has a better skin feeling than possible up to now.

EXAMPLES

The present invention will now be further illustrated in detail by, but is by no means limited to, the following Examples, wherein the amounts are all % by weight unless otherwise noted.

Examples 1 to 3 and Comparative Examples 1 to 3

Stick-type water-in-oil type emulsion cosmetic compositions were prepared by the formulations shown in the following Table I and were evaluated for usability and hardness by the methods explained above. The results are shown in Table II.

1) Evaluation of Usability

Samples of the stick type water-in-oil type emulsion cosmetic compositions were evaluated by a panel of 20 for feeling of use (i.e., softness to the skin, refreshing feeling, freshness after finish, lightness of slip) by directly applying the stick-type water-in-oil type emulsion cosmetic composition to the skin from the stick container.

Evaluation Criteria

G (good): At least 15 panelists responded "good"
F (fair): 7 to 14 panelists responded "good"
P (poor): 6 or less panelists responded "good"

2) Hardness

The hardness was measured by a card tension meter under conditions of 37°C, 200 g load, and 1.5ϕ.

Table I

| | Examples | | | Comparative Examples | | |
|---|---|---|---|---|---|---|
| | 1 | 2 | 3 | 1 | 2 | 3 |
| Decamethylcyclopentasiloxane | 26.0 | 26.0 | 21.0 | 26.0 | 26.0 | 51.0 |
| Polyoxyalkylene-modified organo polysiloxane*1 | 5.0 | - | 5.0 | 5.0 | - | 5.0 |
| Long-chain alkyl-containing polyoxyalkylene-modified organo polysiloxane*2 | - | 5.0 | - | - | 5.0 | - |
| Ceresine wax | 2.0 | - | 5.0 | 10.0 | 5.0 | 8.0 |
| Hydrogenated jojoba oil*3 | 8.0 | 7.0 | 5.0 | - | - | - |
| Microcrystalline wax | - | 1.0 | - | - | 5.0 | - |
| Carnauba wax | - | 2.0 | - | - | - | 2.0 |
| Silicone-treated talc | 16.0 | 16.0 | 16.0 | 16.0 | 16.0 | 16.0 |
| Silicone-treated titanium dioxide | 10.0 | 10.0 | 10.0 | 10.0 | 10.0 | 10.0 |
| Silicone-treated iron oxide (yellow) | 2.9 | 2.9 | 2.9 | 2.9 | 2.9 | 2.9 |
| Silicone-treated iron oxide (red) | 1.0 | 1.0 | 1.0 | 1.0 | 1.0 | 1.0 |
| Silicone-treated iron oxide (black) | 0.1 | 0.1 | 0.1 | 0.1 | 0.1 | 0.1 |
| Polymethylmethacrylate | 4.0 | 4.0 | 4.0 | 4.0 | 4.0 | 4.0 |
| Ion exchanged water | 20.0 | 20.0 | 30.0 | 20.0 | 20.0 | - |
| Dipropylene glycol | 5.0 | 5.0 | - | 5.0 | 5.0 | - |
| Preservative | q.s. | q.s. | q.s. | q.s. | q.s. | q.s. |
| Fragrance | q.s. | q.s. | q.s. | q.s. | q.s. | q.s. |

*1: A compound of the above general formula (I) where a=3, b=8 to 10, c=0, m=50 to 60, n=0, o=2 to 5, $R^2$=H, molecular weight = about 6000, and polyether-modification ratio = about 20% by weight.

*2: A compound of the above general formula (I) where a=3, b=50, c=60, m=100, n=50, o=15, $R^1=C_{16}H_{33}$, $R^2$=H, molecular weight = about 15,000.

*3: Floraesters 70 (made by Floratech)

Table II

| | Examples | | | Comparative Examples | | |
|---|---|---|---|---|---|---|
| | 1 | 2 | 3 | 1 | 2 | 3 |
| Softness to the skin | G | G | G | P | P | P |
| Refreshing feeling | G | G | G | G | G | P |
| Freshness after finish | G | G | G | G | P | G |
| Light slip | G | G | F | G | G | G |
| Hardness | 18 | 25 | 20 | 15 | 8 | 28 |

The method of production in the following Examples was to heat the oil phase ingredients to 80°C to melt them, then disperse the powder in it, emulsify and disperse the aqueous phase ingredients heated in advance, and fill the result and mixture in a container in the fluid state. Next, this was cooled to obtain the desired product. In all cases, the stability was excellent, there was a refreshing feeling, and the smoothness to the skin was superior.

weight = about 6000, and polyether-modification ratio = about 20 wt%

| | |
|---|---|
| Hydrophobically treated pigment powder | 20.0 |
| Ion exchanged water | 10.0 |
| Glyceryl | 15.0 |
| 1,3-butylene glycol | 2.0 |
| Preservative | q.s. |
| Fragrance | q.s |

Example 4: Stick-Type Emulsion Foundation

| | |
|---|---|
| Decamethylcyclopentanesiloxane | 35.0 wt% |
| Polyoxyalkylene-modified organo polysiloxane (compound of above general formula (I) where a=3, b=50, c=60, m=100, n=50, o=15, $R^1=C_{16}H_{33}$, $R^2$=H, molecular weight = about 15,000) | 2.0 |
| Sorbitan sesquiisostearate | 2.0 |
| Ceresine | 2.0 |
| Hydrogenated jojoba oil | 7.0 |
| Hydrophobically treated pigment powder | 15.0 |
| Polyacrylic acid alkyl | 12.0 |
| Dipropylene glycol | 5.0 |
| Ion exchanged water | 20.0 |

(continued)

| | |
|---|---|
| Preservative | q.s. |
| Antioxidants | q.s |

Example 5: Urea-Containing Solid Cosmetic Composition

| | |
|---|---|
| Trimethylsiloxysilicate | 3.0 w |
| Dimethyl polysiloxane | 10.0 |
| Decamethylcyclopentasiloxane | 20.0 |
| Hydrogenated jojoba oil | 8.0 |
| Ceresine | 2.0 |
| Polyoxyalkylene-modified organo polysiloxane (compound of above general formula (I) where a=3, b=24, c=24, m=400, n=0, o=10, $R^2$=H, molecular weight = about 55,000) | 2.0 |
| Ion exchanged water | 46.0 |
| Urea | 3.0 |
| Glyceryl | 3.0 |
| Diglyceryl | 2.0 |
| Propylene glycol | 1.0 |
| Preservative | q.s. |

Example 6: Rouge

| | |
|---|---|
| Dimethyl polysiloxane (1.5 cs) | 20.0 wt% |
| Decamethylcyclopentasiloxane | 10.0 |
| Cetyl isooctanate | 15.0 |
| Polyoxyalkylene-modified organo polysiloxane (compound of above general formula (I) where a=3, b=8 to 10, c=0, m=50 to 60, n=0, o=2 to 5, $R^2$=H, molecular weight = about 6000, polyether modification ratio = about 20 wt% | 3.0 |
| Hydrogenated jojoba oil | 10.0 |
| Hydrocarbon wax | 2.0 |
| Fragrance | q.s. |
| Hydrophobically treated pigment powder | 30.0 |
| Ion exchanged water | 6.9 |
| Sodium hyaluronate | 0.1 |
| Sodium chondroitin sulfate | 1.5 |
| Polyethylene glycol | 1.5 |
| Preservative | q.s. |

Example 7: Lipstick

| | |
|---|---|
| Octamethylcyclotetrasiloxane | 10.0 wt% |
| Dimethyl polysiloxane (6 cs) | 23.0 |
| Carnauba wax | 2.8 |
| Hydrogenated jojoba oil | 8.0 |
| Polyoxyalkylene-modified organo polysiloxane (compound of above general formula (I) where a=3, b=50, c=60, m=100, n=50, o=15, $R^1$=$C_{16}H_{33}$, $R^2$=H, molecular weight = about 15,000) | 5.0 |
| Red iron oxide | 0.3 |

(continued)

| | |
|---|---|
| Yellow iron oxide | 1.0 |
| Lake red CBA (Red No. 204) | 0.7 |
| Dibutyl hydroxytoluene | q.s. |
| Fragrance | q.s. |
| Ion exchanged water | 48.4 |
| Water-soluble collagen solution | 0.3 |
| Sodium pyrrolidonecarboxylate | 0.5 |

Example 8: Lip Treatment

| | |
|---|---|
| Squalane | 10.0 wt% |
| Lanolin | 2.0 |
| Octamethylcyclotetrasiloxane | 27.69 |
| Isoparaffin (boiling point 155°C) | 10.0 |
| Trimethylsiloxysilicate | 3.0 |
| Hydrogenated jojoba oil | 8.0 |
| Polyoxyalkylene-modified organo siloxane (compound of above general formula (I) where a=3, b=24, c=24, m=400, n=0, o=10, $R^2$=H, molecular weight = about 55,000) | 3.0 |
| Ion exchanged water | 30.0 |
| Glyceryl | 5.0 |
| Sodium lactate | 0.3 |
| Sodium L-glutamate | 0.3 |
| Sodium hyaluronate | 0.1 |
| Sorbitol | 0.5 |
| Lithol rubine BCA (Red No. 202) | 0.01 |
| Menthol | 0.1 |
| Fragrance | q.s. |

As explained above, the stick-type solid water-in-oil type emulsion cosmetic composition of the present invention has an excellent stability, has a refreshing feeling and freshness after finish when applied to the skin despite being in solid form, and particularly gives a soft feeling of use when applied to the skin.

## Claims

**1.** A stick-type solid water-in-oil type emulsion cosmetic composition comprising the following components (a) to (c):

(a) 0.5 to 40.0% by weight of a hydrogenated jojoba oil;
(b) 0.1 to 10.0% by weight of a surfactant composed of at least one polyoxyalkylene-modified silicone or alkyl- and polyoxyalkylene-modified silicone having the formula (I):

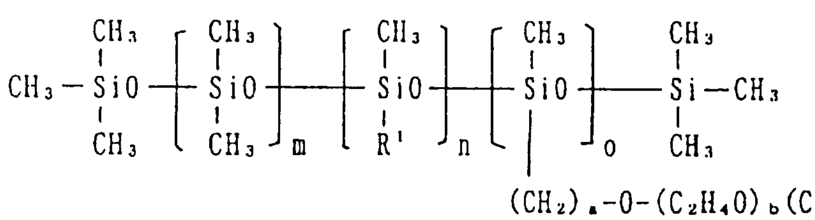

··· (I)

wherein a is an integer of 1 to 5, b is an integer of 7 to 100, c is an integer of 0 to 100, m is an integer of 10

to 500, n is an integer of 0 to 150, and o is an integer of 1 to 50, $R^1$ is a $C_1$ to $C_{26}$ alkyl group, and $R^2$ is a hydrogen atom or a $C_1$ to $C_5$ alkyl group is contained; and
(c) 5.0 to 50.0% by weight of water.

**2.** A stick-type solid water-in-oil type emulsion cosmetic composition as claimed in claim 1, further comprising a hydrocarbon wax, wherein a weight ratio of the hydrocarbon wax/the hydrogenated jojoba oil is 0.05 to 15.

**3.** A stick-type solid water-in-oil type emulsion cosmetic composition as claimed in claim 2, wherein the hydrocarbon wax is ceresine wax.